(19) Europäisches Patentamt
European Patent Office
Office européen des brevets

(11) **EP 2 813 490 B1**

(12) # EUROPÄISCHE PATENTSCHRIFT

(45) Veröffentlichungstag und Bekanntmachung des Hinweises auf die Patenterteilung:
**30.11.2016 Patentblatt 2016/48**

(51) Int Cl.:
***C07C 319/18*** *(2006.01)*   ***C07C 319/26*** *(2006.01)*
***C07C 323/22*** *(2006.01)*

(21) Anmeldenummer: **14169638.5**

(22) Anmeldetag: **23.05.2014**

(54) **Verfahren zur Herstellung von lagerstabilem 3-Methylmercaptopropionaldehyd**

Method for the preparation of storage-stable 3-methylmercaptopropionaldehyde

Procédé de fabrication de 3-méthylmercaptopropionaldéhyde stable au stockage

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.06.2013 EP 13171394**

(43) Veröffentlichungstag der Anmeldung:
**17.12.2014 Patentblatt 2014/51**

(73) Patentinhaber: **Evonik Degussa GmbH 45128 Essen (DE)**

(72) Erfinder:
• **Geiger, Friedhelm verstorben (DE)**

• **Müller, Klaus Peter verstorben (DE)**
• **Theissen, Ferdinand 53332 Bornheim (DE)**

(56) Entgegenhaltungen:
**EP-A1- 0 899 258    WO-A1-93/13059
DE-A1- 2 158 616**

• **J. R. CATCH ET AL: "320. Syntheses of some amino-acids, including methionine", JOURNAL OF THE CHEMICAL SOCIETY (RESUMED), 1. Januar 1947 (1947-01-01), Seite 1609, XP055085742, ISSN: 0368-1769, DOI: 10.1039/jr9470001609**

EP 2 813 490 B1

**Beschreibung**

Die Erfindung betrifft ein Verfahren zur Herstellung von 3-Methylmercaptopropionaldehyd durch Umsetzung von Acrolein mit Methylmercaptan, wobei Verunreinigungen und Nebenprodukte aus der Umsetzung von Acrolein mit Methylmercaptan durch Destillation entfernt werden.

Aus der DE 21 58 616 ist bekannt, dass 3-Methylmercaptopropionaldehyd (MMP) aus Acrolein und Methylmercaptan durch Katalyse mit organischen Stickstoffbasen unter Zusatz von Pyridin und/oder Essigsäure hergestellt wird. Dabei fällt ein roher 3-Methylmercaptopropionaldehyd an, der Verunreinigungen enthält, die sich aus nicht umgesetzten Anteilen der Ausgangssubstanzen sowie Neben- und Spaltprodukte zusammensetzen.

Bei der Anschließenden Destillation des Rohproduktes fallen in erheblichem Maße Polymerisationen und andere Nebenreaktionen auf. Zur Lösung dieses Problems wird in der DE 21 58 616 vorgeschlagen, die Destillation in Anwesenheit eines Gemisches einer schwer flüchtigen starken mit einer leicht flüchtigen schwachen Säure durchzuführen. Im Ergebnis wird das 3-Methylmercaptopropionaldehyd in einer Reinheit von 99,5% erhalten.

Bei der Destillation kann jedoch weder Pyridin noch Essigsäure vollständig zurückgehalten werden. Wie Stabilitätsuntersuchungen zeigten, führen diese Stoffe auch in kleinen Mengen dazu, dass das MMP nicht lagerstabil ist.

Es verändert sich schon nach sehr kurzer Zeit und bildet unerwünschte höhermolekulare Nebenprodukte. Diese stören die Weiterverarbeitung zu Hydantoin und schließlich Methionin erheblich. Das gilt vor allem für das Kaliumcarbonatverfahren, da sich durch die Kreislaufarbeitsweise die Nebenprodukte anreichern und zu erhöhter Ausschleusung führen.

Die EP 0 899 258 offenbart lagerstabiles 3-Methylmercaptopropionaldehyd mit einem Wassergehalt von > 300 ppm sowie ein Verfahren zur Verbesserung der

Lagerstabilität von 3-Methylmercaptopropionaldehyd. Es ist bekannt, dass 3-Methylmercaptopropionaldehyd sehr reaktionsfreudig ist, und dass schon bei der Lagerung Veränderungen eintreten.

Durch die Bildung von Oligomeren, Polymeren und Kondensationsprodukten sowie Eliminierungsreaktionen und Oxidation und den Folgereaktionen der gebildeten Folgeprodukte entsteht eine Vielzahl von unerwünschten Verunreinigungen.

In saurem Umfeld wird die Bildung von Oligomeren, Polymeren und vor allem cyclischen Trimeren (Trioxanen) begünstigt, in alkalischem Umfeld wird die Aldolkondensation begünstigt. So benutzt man gemäss DE-OS 2 905 267 in einem Verfahren zur Stabilisierung von Aldehyden vor cyclischer Trimerisation, Polymerisation und Autokondensation Triethanolamin oder N,N-Dimethylethanolamin und erzeugt so ein alkalisches Umfeld. Ebenso werden in den japanischen Patenten JP 72 321, 963 und JP 49116017 alkylierte Aniline zum gleichen Zweck eingesetzt. Die US-PS 4,546,205 betrifft die Verwendung eines Gemischs aus einem Pyridin und einer Phenolkomponente.

Die genannten Stabilisatoren erzeugen ein basisches Umfeld und verringern die Geschwindigkeit der sauer katalysierten Reaktionen. Die Stabilisatoren sind allerdings teilweise toxisch und werden zum Teil in hoher Dosierung, vorzugsweise bis zu 0,2 % eingesetzt. Aufgrund der Siedepunkte und der Produkteigenschaften ist eine destillative Abtrennung oft nicht möglich.

In der PCT WO 93/13059 wird die Stabilisierung von schwefel-substituierten Alkanalen mit einer Mischung aus einer Aminkomponente zusammen mit einer sauerstoffbindenden Komponente, wie Phenolen, sauren oder ungesättigten Antioxydantien wie Ascorbinsäure oder beta-Carotin beschrieben. Bei diesem Verfahren wird jedoch darauf verwiesen, dass es nur wirksam ist, wenn der Wassergehalt des Alkanals unter 300 ppm liegt, vorzugsweise unter 100 ppm. Gemäß EP 0 899 258 wird lagerstabiles 3-Methylmercaptopropionaldehyd durch Zusatz einer organischen, gesättigten, Metallkomplexe bildende Säure erhalten. Bei den genannten sauren Verbindungen handelt es sich um solche Verbindungen, die aufgrund ihrer Struktur in der Lage sind, Metalle zu komplexieren, insbesondere Hydroxydicarbonsäuren wie z. H. Zitronensäure, Weinsäure oder Äpfelsäure, insbesondere auch Aminotrismethylenphosphonsäure (ATMP). Nachteilig bei dieser Vorgehensweise ist jedoch, dass zur Erreichung der Lagerstabilität dem 3-Methylmercaptopropionaldehyd ein Additiv zugegeben werden muss, das potentiell die Weiterverarbeitung stört und eine erneute Reinigung nach der Lagerung erforderlich macht.

Aufgabe der vorliegenden Erfindung ist es daher Verfahren zur Herstellung von 3-Methylmercaptopropionaldehyd zur Verfügung zu stellen, bei dem die Nachteile des Standes der Technik zumindest verringert sind.

Diese und weitere Aufgaben werden erfindungsgemäß gelöst durch ein Verfahren zur Herstellung von 3-Methylmercaptopropionaldehyd durch Umsetzung von Acrolein mit Methylmercaptan,

wobei Verunreinigungen und Nebenprodukte aus der Umsetzung von Acrolein mit Methylmercaptan durch Destillation entfernt werden,

dadurch gekennzeichnet, dass die Umsetzung in Gegenwart eines basischen Amins durchgeführt wird, dass das basische Amin einen $pK_b$ kleiner 8 aufweist,

dass vor der Destillation, bzw. nach der Umsetzungsreaktion, das basische Amin durch Zugabe einer Säure neutralisiert wird

und dass die Säure einen $pK_s$ kleiner 5 und einen Dampfdruck kleiner 10 hPa bei 20 °C aufweist.

Völlig überraschend konnte gezeigt werden, dass durch die Verwendung eines basischen Amins mit einem $pK_b$ kleiner 8 anstelle von Pyridin für die Umsetzung von Acrolein mit Methylmercaptan, ein lagerstabiles 3-Methylmercaptopropionaldehyd erhalten wird, welches nicht durch Zugabe von Additiven stabilisiert werden muss. Das durch das erfindungsgemäße Verfahren erhaltene 3-Methylmercaptopropionaldehyd zeichnet sich bevorzugt dadurch aus, dass der Gehalt an 3-Methylmercaptopropionaldehyd um weniger als 0,8%, bevorzugt weniger als 0,5%, bzw. weniger als 0,3% nach 5 Tagen bei Lagerung in einem lichtundurchlässigen Behälter unter Raumtemperatur im Vergleich zum 3-Methylmercaptopropionaldehyd-Gehalt vor der Lagerung abnimmt.

Besonders bevorzugt nimmt der 3-Methylmercaptopropionaldehyd-Gehalt in 10 Tagen um weniger als 0,8%, insbesondere weniger als 0,5%, bzw. weniger als 0,3% ab.

Die Bestimmung des Gehaltes an 3-Methylmercaptopropionaldehyd ist dem Fachmann bekannt und kann bevorzugt gaschromatographisch ermittelt werden.

Der $pK_b$ Wert ist definiert als der negative dekadische Logarithmus der Basenkonstante ($K_b$) in Analogie zum $pK_s$ Wert, der definiert ist als der negative dekadische Logarithmus der Säurekonstante ($K_s$).

Die Basenkonstante ($K_b$) ist eine Größe, welche angibt, in welchem Maße eine Base in einem Lösungsmittel alkalisch reagiert. Die Basenkonstante ($K_b$) lässt sich direkt aus der Säurekonstante ($K_s$) berechnen.

$$K_s \cdot K_b = 10^{-14}$$

$$pK_b = -\log K_b$$

$$pK_s + pK_b = 14$$

Es gilt dabei, dass je kleiner der $pK_b$-Wert, desto stärker das Bestreben der Base, Protonen aufzunehmen.

Erfindungsgemäß ist es erforderlichen, dass die Umsetzung von Acrolein mit Methylmercaptan in Gegenwart eines basischen Amins mit einem $pK_b$ kleiner 8 stattfindet. Das im Stand der Technik verwendete Pyridin weist einen $pK_b$ Wert von 8,94 auf. Pyridin ist daher als Katalysator für das erfindungsgemäße Verfahren zur Herstellung von 3-Methylmercaptopropionaldehyd durch Umsetzung von Acrolein mit Methylmercaptan ungeeignet.

In bevorzugten Ausführungsformen zeichnet sich das erfindungsgemäße Verfahren dadurch aus, dass das basische Amin einen $pK_b$ kleiner 7, einen $pK_b$ kleiner 6,5, einen $pK_b$ kleiner 6, einen $pK_b$ kleiner 5 und besonders bevorzugt einen $pK_b$ kleiner 4 aufweist.

Eine weitere bevorzugte Ausführungsform des erfindungsgemäßen Verfahrens ist dadurch gekennzeichnet, dass das basische Amin ein Alkylamin ist, wobei dieses Alkylamin sich selbstverständlich auch durch einen $pK_b$ kleiner 8, einen $pK_b$ kleiner 7, einen $pK_b$ kleiner 6,5, einen $pK_b$ kleiner 6, einen $pK_b$ kleiner 5 und besonders bevorzugt einen $pK_b$ kleiner 4 auszeichnen muss.

Besonders bevorzugt wird das basische Amin ausgewählt aus der Gruppe Triethylamin oder Trimethylamin. Trimethylamin weist einen $pK_b$ von 4,24, Triethylamin einen $pK_b$ von 3,25 auf.

Erfindungsgemäß wird vor der Aufreinigung durch Destillation, bzw. nach der Umsetzungsreaktion, das basische Amin durch Zugabe einer Säure neutralisiert.

Zur Erreichung der Lagerstabilität des im erfindungsgemäßen Verfahren hergestellten 3-Methylmercaptopropionaldehyd ist es erforderlich, dass die Säure zur Neutralisation des basischen Amins eine starke, nicht flüchtige Säure ist.

Unter einer starken, nicht flüchtigen Säure im Sinne der vorliegenden Erfindung ist eine Säure zu verstehen, die einen $pK_s$ kleiner 5 und einen Dampfdruck kleiner 10 hPa bei 20 °C aufweist.

Das erfindungsgemäße Verfahren zeichnet sich daher dadurch aus, dass die Säure, die vor der Destillation zur Neutralisation des basischen Amins zugegeben wird, einen $pK_s$ kleiner 5 und einen Dampfdruck kleiner 10 hPa bei 20 °C aufweist. Insbesondere weist die Säure einen $pK_s$ kleiner 3 und einen Dampfdruck kleiner 5 hPa bei 20 °C auf

Besonders bevorzugt, wird die Säure ausgewählt wird aus der Gruppe Schwefelsäure oder Phosphorsäure.

Der $pK_s$ Wert der Schwefelsäure beträgt -3, der Dampfdruck bei 20°C beträgt 1,3 hPa. Der $pK_s$ Wert der Posphorsäure beträgt 2,16, der Dampfdruck bei 20°C beträgt 3,8 Pa.

Das basische Amin kann daher nicht mit Salzsäure ($pk_s$ -7, Dampfdruck 190 hPa bei 20°C) neutralisiert werden, da sich für den Fall, dass die Destillation als Vakuumdestillation durchgeführt wird, Chlorwasserstoff bilden kann.

Auch ist die Neutralisation des basischen Amins mit Essigsäure ($pk_s$ 4,76, Dampfdruck 16 hPa bei 20°C) nachteilig, da die Gegenwart von Essigsäure zur Bildung höhermolekularer Nebenprodukte führt.

Die Konzentration der Säure zur Neutralisation des basischen Amins liegt bevorzugt im Bereich von 1N - 2N.

Weiterhin ist das erfindungsgemäße Verfahren bevorzugt dadurch gekennzeichnet, dass die Säure in Bezug auf das basische Amin mindestens stöchiometrisch zugegeben wird. Besonders bevorzugt wird die Säure in einem Überschuss von 0,05% bis 0,1% zugegeben.

Nach den besonders bevorzugten Ausführungsformen des erfindungsgemäßen Verfahrens gelingt es, die Nachteile des Standes der Technik vollständig zu vermeiden und ein völlig lagerstabiles 3-Methylmercaptopropionaldehyd zu erhalten. Die Destillation kann, wie in der DE 21 58 616 offenbart, zweistufig vorgenommen werden. Diese aufwendige Betriebsweise ist nach dem erfindungsgemäßen Verfahren allerdings nicht mehr nötig. Vielmehr kann 3-Methylmercaptopropionaldehyd auch durch einmalige Destillation, bevorzugt durch eine einmalige Vakuumdestillation, in lagerstabiler Qualität gewonnen werden.

Wie die folgenden Beispiele zeigen, ist das nach dem erfindungsgemäßen Verfahren gewonnene 3-Methylmercaptopropionaldehyd lagerstabil und verändert sich auch nach einem längeren Transport nicht.

Vergleichsbeispiel 1

3-Methylmercaptopropionaldehyd, hergestellt erfindungsgemäß durch Umsetzung von Acrolein mit Methylmercaptan in Gegenwart von Triethylamin, anschließender Neutralisation mit Schwefelsäure und darauffolgender Vakuumdestillation wurde mit 1 ppm Pyridin versetzt, um die nach dem Stand der Technik enthaltenen Verunreinigungen zu simulieren. Das so hergestellte 3-Methylmercaptopropionaldehyd wurde unter Lichtabschluss bei Raumtemperatur 5 Tage lang gelagert. Anschließend wurde gaschromatographisch der MMP-Gehalt ermittelt. Die Reinheit hat um 1 % abgenommen.

Vergleichsbeispiel 2

3-Methylmercaptopropionaldehyd hergestellt wie in Vergleichsbeispiel 1 beschrieben, wurde anstelle mit 1 ppm Pyridin mit 1 ppm Essigsäure versetzt. Das so hergestellte 3-Methylmercaptopropionaldehyd wurde unter Lichtabschluss bei Raumtemperatur 5 Tage lang gelagert. Anschließend wurde gaschromatographisch der MMP-Gehalt ermittelt. Die Reinheit hat um 1,2 % abgenommen.

Vergleichsbeispiel 3

3-Methylmercaptopropionaldehyd hergestellt wie in Vergleichsbeispiel 1 beschrieben, wurde anstelle mit 1 ppm Pyridin mit je 1 ppm Pyridin und 1 ppm Essigsäure versetzt und über 8 Tage unter Lichtabschluss bei Raumtemperatur gelagert. Die Abnahme der Reinheit betrug 1,3 %.

Beispiel 1

3-Methylmercaptopropionaldehyd, hergestellt erfindungsgemäß durch Umsetzung von Acrolein mit Methylmercaptan in Gegenwart von Triethylamin, anschließender Neutralisation mit Schwefelsäure und darauffolgender Vakuumdestillation wurde ohne Zugabe von Pyridin oder Essigsäure und ohne Zugabe anderer Additive unter Lichtabschluss bei Raumtemperatur über 10 Tage gelagert. Nach dieser Zeit ist keine Abnahme der Reinheit festzustellen.

Beispiel 2

3-Methylmercaptopropionaldehyd, großtechnisch hergestellt wie unter Beispiel 1 beschrieben, wird im Kesselwagen bei Umgebungstemperatur über eine Woche transportiert. Bei Ankunft hat sich die Reinheit des 3-Methylmercaptopropionaldehyd nicht verändert. Es kann ohne Redestillation direkt weiterverarbeitet werden.

**Patentansprüche**

1. Verfahren zur Herstellung von 3-Methylmercaptopropionaldehyd durch Umsetzung von Acrolein mit Methylmercaptan,
   wobei Verunreinigungen und Nebenprodukte aus der Umsetzung von Acrolein mit Methylmercaptan durch Destillation entfernt werden, **dadurch gekennzeichnet,**
   **dass** die Umsetzung in Gegenwart eines basischen Amins durchgeführt wird, dass das basische Amin einen $pK_b$ kleiner 8 aufweist,
   **dass** vor der Destillation das basische Amin durch Zugabe einer Säure neutralisiert wird
   und **dass** die Säure einen $pK_s$ kleiner 5 und einen Dampfdruck kleiner 10 hPa bei 20 °C aufweist.

2. Verfahren nach Anspruch 1, **dadurch gekennzeichnet, dass** das basische Amin einen $pK_b$ kleiner 6,5 aufweist.

3. Verfahren nach Anspruch 2, **dadurch gekennzeichnet, dass** das basische Amin einen $pK_b$ kleiner 5 aufweist.

4. Verfahren nach einem der Ansprüche 1 bis 3, **dadurch gekennzeichnet, dass** das basische Amin ein Alkylamin ist.

5. Verfahren nach Anspruch 4, **dadurch gekennzeichnet, dass** das basische Amin ausgewählt wird aus der Gruppe Triethylamin oder Trimethylamin.

6. Verfahren nach einem der Ansprüche 1 bis 5, **dadurch gekennzeichnet, dass** die Säure in Bezug auf das basische Amin mindestens stöchiometrisch zugegeben wird.

7. Verfahren nach Anspruch 6, **dadurch gekennzeichnet, dass** die Säure in einem Überschuss von 0,05% bis 0,1% zugegeben wird.

8. Verfahren nach einem der Ansprüche 1 bis 7, **dadurch gekennzeichnet, dass** die Säure ausgewählt wird aus der Gruppe Schwefelsäure oder Phosphorsäure.

**Claims**

1. Process for preparing 3-methylmercaptopropionaldehyde by reacting acrolein with methyl mercaptan, wherein impurities and by-products from the reaction of acrolein with methyl mercaptan are removed by distillation, **characterized in that** the reaction is carried out in the presence of a basic amine, the basic amine has a $pK_b$ less than 8, prior to the distillation the basic amine is neutralized by addition of an acid and the acid has a $pK_a$ less than 5 and a vapour pressure less than 10 hPa at 20°C.

2. Process according to Claim 1, **characterized in that** the basic amine has a $pK_b$ less than 6.5.

3. Process according to Claim 2, **characterized in that** the basic amine has a $pK_b$ less than 5.

4. Process according to any one of Claims 1 to 3, **characterized in that** the basic amine is an alkylamine.

5. Process according to Claim 4, **characterized in that** the basic amine is selected from the group of triethylamine or trimethylamine.

6. Process according to any of Claims 1 to 5, **characterized in that** the acid is added at least stoichiometrically relative to the basic amine.

7. Process according to Claim 6, **characterized in that** the acid is added in an excess of from 0.05% to 0.1%.

8. Process according to any of Claims 1 to 7, **characterized in that** the acid is selected from the group of sulphuric acid or phosphoric acid.

**Revendications**

1. Procédé pour la préparation de 3-méthylmercaptopropionaldéhyde par transformation d'acroléine avec du méthylmercaptan, les impuretés et les produits secondaires de la transformation d'acroléine avec du méthylmercaptan étant éliminées par distillation, **caractérisé en ce que** la transformation est réalisée en présence d'une amine basique, **en ce que** l'amine basique présente un $pK_b$ inférieur à 8, **en ce que** l'amine basique est neutralisée avant la distillation par addition d'un acide et **en ce que** l'acide présente un $pK_a$ inférieur à 5 et une tension de vapeur inférieure à 10 hPa à 20°C.

2. Procédé selon la revendication 1, **caractérisé en ce que** l'amine basique présente un $pK_b$ inférieur à 6,5.

3. Procédé selon la revendication 2, **caractérisé en ce que** l'amine basique présente un $pK_b$ inférieur à 5.

4. Procédé selon l'une quelconque des revendications 1 à 3, **caractérisé en ce que** l'amine basique est une alkylamine.

5. Procédé selon la revendication 4, **caractérisé en ce que** l'amine basique est choisie dans le groupe formé par la triéthylamine ou la triméthylamine.

6. Procédé selon l'une quelconque des revendications 1 à 5, **caractérisé en ce que** l'acide est ajouté en une quantité au moins stoechiométrique par rapport à l'amine basique.

7. Procédé selon la revendication 6, **caractérisé en ce que** l'acide est ajouté en un excès de 0,05% à 0,1%.

8. Procédé selon l'une quelconque des revendications 1 à 7, **caractérisé en ce que** l'acide est choisi dans le groupe formé par l'acide sulfurique ou l'acide phosphorique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2158616
- EP 0899258 A
- DE OS2905267 A
- JP 47321963 A
- JP 49116017 B
- US 4546205 A
- WO 9313059 A